# EUROPÄISCHE PATENTSCHRIFT

(11) **EP 0 782 421 B1**
(45) Veröffentlichungstag und Bekanntmachung des Hinweises auf die Patenterteilung: **14.07.1999**
(21) Anmeldenummer: 95931891.6
(22) Anmeldetag: 18.09.1995
(51) Int. Cl.: A61F 13/02, A61B 19/00

(54) **VORRICHTUNG ZUM VERSIEGELN EINES WUNDGEBIETES**
DEVICE FOR SEALING OFF AN INJURY AREA
DISPOSITIF PERMETTANT DE RENDRE ETANCHE LA ZONE D'UNE PLAIE

(30) Priorität: 20.09.1994 DE 4433450
(43) Veröffentlichungstag der Anmeldung: 09.07.1997
(73) Patentinhaber: Fleischmann, Wim, 74321 Bietigheim-Bissingen (DE)
(72) Erfinder: Fleischmann, Wim, 74321 Bietigheim-Bissingen (DE)
(74) Vertreter: Patentanwälte Westphal, Mussgnug & Partner
(86) Internationale Anmeldenummer: DE9501292
(87) Internationale Veröffentlichungsnummer: WO9609022

(56) Entgegenhaltungen:
- WO-A-89/02259
- WO-A-90/11795
- WO-A-93/09727
- DE-A- 3 443 101
- FR-A- 2 393 566
- US-A- 3 805 789
- US-A- 3 954 105
- US-A- 4 969 881

## Beschreibung

Die Erfindung bezieht sich auf eine Vorrichtung zum Abdecken eines Wundgebietes gemäß dem Oberbegriff des Anspruchs 1.

Eine Vorrichtung dieser Gattung ist aus der GB-A-1, 280, 631 zum Abdecken von Wunden bekannt. Eine Folie überdeckt das Wundgebiet und wird rings um die Wundränder auf die Haut geklebt. Die Folie ist dampfdurchlässig, um eine Ansammlung von Wundsekret unter der Folie zu vermeiden. Die Folie und der Klebstoff zur Befestigung der Folie sind wasserfest und verhindern das Eindringen von Flüssigkeiten und Mikroorganismen. Ein luftdichtes Verschließen des Wundgebietes durch die Folie ist nicht beabsichtigt.

Aus der WO-A-90/11 795 ist es bekannt, das Wundgebiet mit einer flüssigkeitsdichten, luftdurchlässigen Folie abzudecken und das Wundsekret über eine durch die Folie geführte Drainage abzusaugen. Die Folie wird mit ihrem Rand rings um das Wundgebiet auf die Hautoberfläche geklebt. Es ist dabei auch vorgesehen, zwischen der Wundfläche und der Folie eine Zwischenschicht aus einem Hydrokolloid anzuordnen. Die auf der Wundfläche aufliegende Hydrokolloid-Schicht dient dazu, das Wundsekret zu absorbieren.

Aus der FR-A-2 393 566 und der US-A-3, 805, 789 ist es bekannt, einen künstlichen Darmausgang mit einem Hydrogel abzudichten, welches mittels auf die Hautoberfläche geklebter Folien gehalten wird. Das Hydrogel dient zur Abdichtung des Darmausganges und nicht zur Abdichtung der Folie. Die Folie hat keine Abdichtungsfunktion.

Aus der WO-A-93/09 727 ist es bekannt, Wundgebiete, wie z. B. frische oder chronische Wunden, Verbrennungen, Verätzungen usw. mit einer wasserdampfdurchlässigen, luftundurchlässigen Folie abzudecken. Die Folie wird rings um das Wundgebiet an der Hautoberfläche aufgeklebt, um das Wundgebiet luftdicht abzuschließen. Über eine unter der Folie in das Wundgebiet eingelegte Drainage wird ein Unterdruck im Wundgebiet erzeugt. Insbesondere bei größeren und tieferen Wunden kann in der Wunde eine poröse Schaumstoffeinlage angeordnet werden, in welche die Drainage eingezogen ist, um das Wundgebiet großflächig zu evakuieren und das Wundsekret abzusaugen. Wird bei dieser Vorrichtung die Drainage auf der Hautoberfläche unter der Folie herausgeführt, so entsteht im Bereich der Drainage eine Undichtigkeit zwischen der Hautoberfläche und der Folie, die die Erzeugung oder Aufrechterhaltung des gewünschten Unterdrucks erschwert oder verhindert. Es ist daher üblich, die Drainage von dem Wundgebiet durch das Körpergewebe hindurchzuführen und außerhalb der Folie durch die Hautoberfläche herauszuführen. Diese Methode ist mit einer zusätzlichen Verletzung verbunden, die ein Infektionsrisiko mit sich bringt. Außerdem ist das Durchziehen der Drainage durch das Gewebe nicht ohne Anästhesie möglich, so daß sich das Verfahren wenig für den ambulanten Einsatz und die Notfallversorgung eignet.

Der Erfindung liegt die Aufgabe zugrunde, eine Vorrichtung zum Versiegeln eines Wundgebietes zur Verfügung zu stellen, welche ein einfaches und zuverlässiges luftdichtes Abschließen des Wundgebietes ermöglicht.

Diese Aufgabe wird erfindungsgemäß gelöst durch eine Vorrichtung mit den Merkmalen des Anspruches 1.

Vorteilhafte Ausführungen der Erfindung sind in den Unteransprüchen angegeben.

Der Erfindung liegt der Gedanke zugrunde, ein leicht verformbares, nicht fließendes Dichtungsmaterial zu verwenden, um Undichtigkeiten zwischen der das Wundgebiet abdeckenden Folie und der Hautoberfläche luftdicht zu verschließen. Dieses Dichtungsmaterial, das vorzugsweise ein Silikon, ein Hydrokolloid oder ein Lyogel, insbesondere ein Hydrogel, ist, läßt sich leicht von Hand verformen und den Hautunebenheiten oder eventuellen Falten der Folie anpassen, wobei das Dichtungsmaterial in die vorhandenen Undichtigkeiten eindringt und diese verschließt. Die Verformbarkeit und die Fließeigenschaften des Dichtungsmaterials werden so eingestellt, daß eine leichte Verformbarkeit und ein gutes Eindringen in Undichtigkeiten gewährleistet ist, jedoch ein Abfließen des Dichtungsmaterials unter der Wirkung des Druckgradienten ausgeschlossen ist.

Wird eine Vakuumversiegelung des Wundgebietes vorgenommen, so kann die zum Absaugen verwendete Drainage auf der Hautoberfläche unter der Folie herausgeführt werden, wobei das Dichtungsmaterial die Drainage umschließt und im Bereich der Drainage zwischen der Folie und der Hautoberfläche auftretende Zwischenräume abdichtend ausfüllt.

Bei der Abdeckung großflächiger Areale der Körperoberfläche, wie sie z.B. bei Verbrennungen, bei Unfällen und dgl. insbesondere auch zur ersten Notversorgung wichtig ist, ist es schwierig, die abdeckende Folie faltenfrei und damit dicht auf der Körperoberfläche aufzubringen und anzukleben. Hier bietet das Dichtungsmaterial die Möglichkeit, eine Abdichtung zwischen der Folie und der Hautoberfläche auch bei Unebenheiten der Körperoberfläche und bei Falten der Folie zu bewirken. Das Dichtungsmaterial wird hierzu streifenförmig entlang des abzudichtenden Randes der Folie zwischen der Hautoberfläche und der Folie angebracht. Hierbei kann die Folie insbesondere schlauch- oder sackförmig ausgebildet sein, um beispielsweise eine Extremität des Patienten zu umhüllen. Der zu verschließende Rand der sackförmigen Folie bzw. die beiden zu verschließenden Ränder der schlauchförmigen Folie können mittels eines die Extremität umschließenden bandförmigen Streifens des Dichtungsmaterials abgedichtet werden. GegebenenfallS kann auch in solchen Anwendungsfällen eine Drainage mittels des Dichtungsmaterials abgedichtet unter die Folie geführt werden.

Um die für die Verwendung des Dichtungsmaterials geeignete Einstellung der Verformbarkeit und der Fließeigenschaften zu gewährleisten, ist es erforderlich, den Feuchtigkeitsgehalt des Dichtungsmaterials bei der Lagerung aufrechtzuerhalten. Hierzu kann das Dichtungsmaterial in einer Tube oder Kartusche zur Verfügung gehalten werden, so daß das Dichtungsmaterial bei Bedarf an der gewünschter Stelle aus der Tube oder der Kartusche ausgebracht werden kann. Eine für viele Anwendungen komfortablere Möglichkeit besteht darin, das Dichtungsmaterial in Form von Streifen oder Platten zur Verfügung zu halten, die durch eine Hüllfolie dicht abgeschlossen oder in einem dicht verschlossenen Behälter gelagert werden. Die Hüllfolie kann abziehbar ausgebildet sein, um das Dichtungsmaterial nach dem Abziehen der Hüllfolie aufzubringen. Es ist auch möglich, das Dichtungsmaterial dauerhaft in die Hüllfolie einzuschließen. In diesem Fall kann die Hüllfolie vorzugsweise eine adhäsive Außenbeschichtung aufweisen, so daß das Dichtungsmaterial an der gewünschten Stelle aufgeklebt werden kann. Die Hüllfolie muß dabei so weich und flexibel sein, daß sie die Verformbarkeit des Dichtungsmaterials nicht behindert.

Eine solche Ausführung mit einer das Dichtungsmaterial umschließenden Hüllfolie eignen sich besonders für eine anwendungsfreundliche Konfektionierung. Beispielsweise kann eine Drainage bereits mit dem Dichtungsmaterial umschlossen konfektioniert werden. Die Drainage kann in diesem Fall unter die Folie eingelegt werden, wobei durch das konfektionierte Dichtungsmaterial bereits ein abgedichtetes Herausführen der Drainage unter der Folie gewährleistet ist.

Weiter kann das Dichtungsmaterial mit einer Hüllfolie konfektioniert als bandförmiger Streifen zur Verfügung gestellt werden. Diese Ausführung eignet sich besonders zum Abdichten entlang des Randes einer größerflächigen Folie. Ist in dieser Ausführung die Hüllfolie auf beiden Seiten des bandförmigen Streifens mit einer selbstklebenden Außenbeschichtung versehen, so kann das streifenförmige Dichtungsmaterial nicht nur zum Abdichten des Randes der Folie dienen, sondern gleichzeitig auch zum Festkleben der Folie auf der Hautoberfläche.

Die verwendeten Dichtungsmaterialien sind im allgemeinen hautfreundlich, so daß sie gegebenenfalls auch unmittelbar auf die Haut aufgebracht werden können. Da die Feuchtigkeit des Dichtungsmaterials jedoch bei längerer Einwirkung zu einer nachteiligen Aufweichung der Hautoberfläche führt, ist zu bevorzugen, das Dichtungsmaterial nicht unmittelbar auf die Haut aufzubringen. Ist das Dichtungsmaterial dauerhaft in eine Hüllfolie eingeschlossen, so schützt diese Hüllfolie auch die Haut vor einem Aufweichen. In anderen Fällen wird vorzugsweise eine Schutzfolie auf die Hautoberfläche aufgebracht, die verhindert, daß das feuchte Dichtungsmaterial mit der Haut in Berührung kommt.

Im folgenden wird die Erfindung anhand von in der Zeichnung dargestellten Ausführungsbeispielen näher erläutert. Es zeigen :
- Figur 1: schematisch eine Draufsicht auf die Vorrichtung zum Versiegeln eines Wundgebietes,
- Figur 2: einen Schnitt längs der Linie II-II in Figur 1,
- Figur 3: schematisch das Anbringen des Dichtungsmateriales im Ausführungsbeispiel der Figur 1,
- Figur 4: im Querschnitt eine mit Dichtungsmaterial konfektionierte Drainage und
- Figur 5: im Querschnitt die Vorrichtung zum Versiegeln eines großflächigen Areals einer Körperextremität.

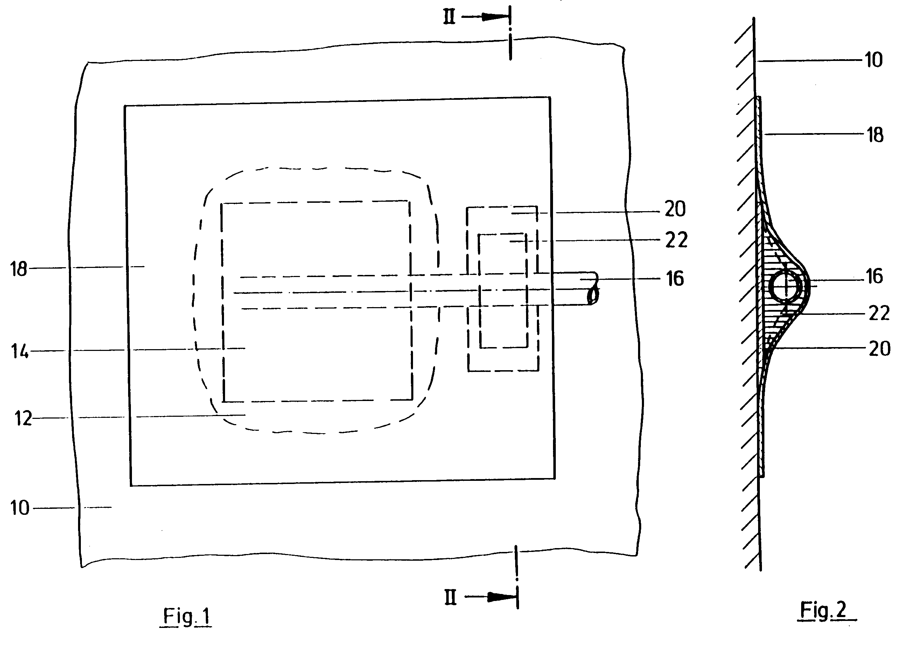

In den Figuren 1 bis 3 ist die Vakuum-Versiegelung eines Wundgebietes dargestellt.

In der Hautoberfläche 10 ist ein Wundgebiet 12 in Form einer großflächigen tiefen Wunde zu behandeln. In das Wundgebiet 12 wird eine poröse Schaumstoffeinlage 14 eingelegt. Eine Drainage (Redon-Drainage) 16 ist mit ihrem gelochten Ende in die Schaumstoffeinlage 14 eingezogen und wird auf der Hautoberfläche 10 aus dem Wundgebiet 12 herausgeführt. Eine wasserdampfdurchlässige, luftundurchlässige Folie 18 wird zur Abdeckung auf das Wundgebiet 12 aufgelegt. Die Folie 18 greift mit ihrem Rand allseitig über den Rand des Wundgebietes 12 und wird mit ihrem Rand rings um das Wundgebiet 12 auf der Hautoberfläche 10 aufgeklebt. Die Drainage 16 wird mit ihrem äußeren Ende an eine Saugpumpe angeschlossen, so daß über die Drainage 16 und die Schaumstoffeinlage 14 in dem Wundgebiet 12 unter der Folie 18 ein Unterdruck erzeugt wird und das Wundsekret über die Poren der Schaumstoffeinlage 14 und die Drainage 16 abgesaugt wird.

In dem Randbereich der Folie 18, in welchem die Drainage 16 zwischen der Hautoberfläche 10 und der Folie 18 herausgeführt wird, kann die Folie 18 nicht abdichtend an der Hautoberfläche 10 anliegend aufgeklebt werden, so daß im Bereich der Drainage 16 zwischen der Hautoberfläche 10 und der Folie 18 Undichtigkeiten auftreten, die die Erzeugung und Aufrechterhaltung des Unterdrucks im Wundbereich 12 erschweren und behindern. Diese Undichtigkeiten werden durch ein Dichtungsmaterial 22 verschlossen, welches die freien Zwischenräume zwischen der Hautoberfläche 10, der Drainage 16 und der Folie 18 ausfüllt. Das Dichtungsmaterial 22 ist ein leicht verformbares, aber nicht fließendes Material, insbesondere ein Silikon, ein Hydrokolloid oder ein Hydrogel. Das Dichtungsmaterial 22 kann aufgrund seiner gallertartigen Konsistenz in die Zwischenräume zwischen Hautoberfläche 10, Drainage 16 und Folie 18 eingedrückt werden und füllt diese Zwischenräume dichtend aus. Das Dichtungsmaterial 22 weist dabei eine ausreichende Formfestigkeit auf, so daß es durch den beim Evakuieren des Wundgebietes 12 zwischen Außenseite und Innenseite der Folie 18 entstehenden Druckgradienten nicht nach innen in das Wundgebiet 12 fließt, sondern in seiner abdichtenden Lage verbleibt.

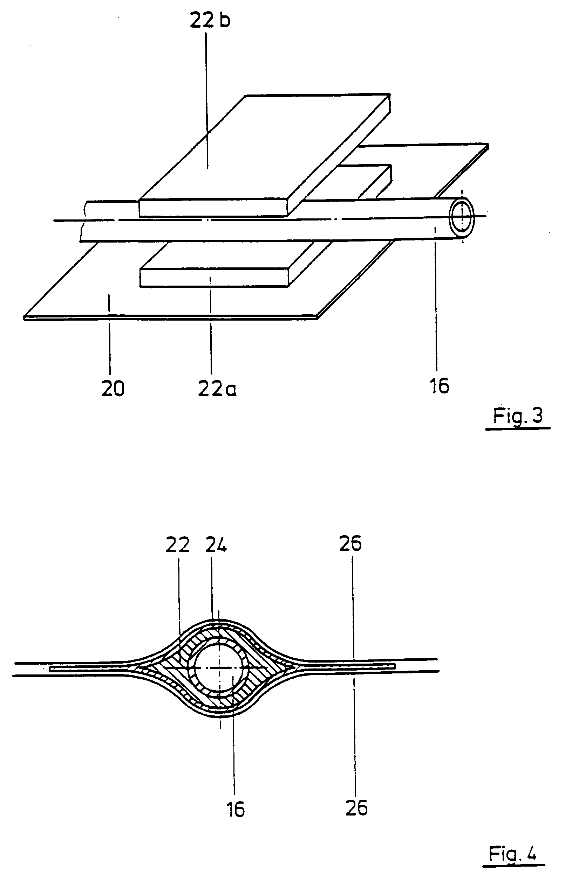

Figur 3 zeigt schematisch das Abdichten der Drainage-Durchführung mittels des Dichtungsmaterials 22 in einer ersten Ausführung. Damit das Feuchtigkeit enthaltende Dichtungsmaterial 22 die Hautoberfläche 10 nicht angreift und aufweicht, wird zunächst in dem Bereich, in welchem die Drainage 16 nach außen geführt wird, eine Schutzfolie 20 auf die Hautoberfläche geklebt. Auf diese Schutzfolie 20 wird eine erste Platte 22a des Dichtungsmaterials 22 aufgelegt. Dann wird die Drainage 16 auf diese erste Platte 22a gelegt und mit einer zweiten Platte 22b des Dichtungsmaterials 22 bedeckt. Die obere Platte 22b wird dann niedergedrückt, so daß sie sich mit der unteren Platte 22a verbindet und das Dichtungsmaterial 22 der beiden Platten 22a und 22b die Drainage 16 vollständig umschließt. Nun kann die Folie 18 aufgebracht und aufgeklebt werden. Die Folie 18 wird dabei auf das die Drainage 16 umschließende Dichtungsmaterial 22 gedrückt, so daß keine Undichtigkeit mehr verbleibt, wie dies in Figur 2 ersichtlich ist.

Damit das Dichtungsmaterial 22 die geeignete Konsistenz und Feuchtigkeit behält, werden die Platten 22a, b des Dichtungsmaterials 22 unter Luftabschluß gelagert und zur Verfügung gehalten. Die Platten 22a, b des Dichtungsmaterials 22 können hierzu entweder in luftdicht geschlossenen Behältern gelagert werden oder in einer Hüllfolie luftdicht eingeschlossen sein, die vor Gebrauch abgezogen wird.

Figur 4 zeigt eine weitere Ausführung, bei welcher die Drainage 16 bereits mit dem Dichtungsmaterial 22 konfektioniert ist. In einem entsprechenden Abstand von dem gelochten Ende der Drainage 16 ist die Drainage 16 von einer ausreichenden Menge des Dichtungsmaterials 22 umschlossen. Das Dichtungsmaterial 22 ist von einer Hüllfolie 24 ummantelt, die auf ihrer Außenfläche mit einer selbstklebenden Außenbeschichtung versehen ist. Um die adhäsive Hüllfolie 24 während der Lagerung zu schützen, ist diese beidseitig durch eine abziehbare Deckfolie 26 geschützt.

Nach dem Positionieren der Drainage 16 in dem Wundgebiet 12 wird die Deckfolie 26 auf der Unterseite abgezogen und die Hüllfolie 24 wird zur Fixierung der Drainage 16 auf die Hautoberfläche 10 geklebt. Anschließend wird die obere Deckfolie 26 abgezogen. Nun kann die Folie 18 angebracht werden, wobei sie im Bereich des DichtungsmaterialS 22 an der adhäsiven Deckfolie 26 festklebt. Das Dichtungsmaterial 22 wird durch Andrücken so verformt, daß zwischen der Folie 18 und der Hautoberfläche 10 keine Undichtigkeiten mehr verbleiben.

Zum Abdecken und Versiegeln größerer Areale der Körperoberfläche kann sich das unmittelbare Aufkleben der Folie 18 auf die Hautoberfläche 10 als unzweckmäßig erweisen, da Falten und Wölbungen der Hautoberfläche 10 ein dichtes Anliegen des Randes der Folie 18 erschweren und behindern. Für solche Anwendungen wird das Dichtungsmaterial 22 als bandförmiger Streifen entlang des gesamten Randes der Folie 18 zwischen die Hautoberfläche 10 und die Folie 18 gebracht, um die Undichtigkeiten zu verschließen, die durch Faltenbildung der Folie 18 entstehen.

Vorzugsweise wird das Dichtungsmaterial 22 dabei als bandförmiger Streifen in einer beidseitig adhäsiv beschichteten Hüllfolie 24 konfektioniert verwendet, wobei die adhäsive Beschichtung der Hüllfolie 24 durch eine abziehbare Deckfolie 26 geschützt ist.

In dieser Ausführung kann der selbstklebende Streifen mit dem Dichtungsmaterial 22 zunächst auf die Hautoberfläche 10 aufgeklebt werden und anschließend wird die Folie 18 aufgebracht, die ihrerseits mittels des selbstklebenden Streifens des Dichtungsmaterials 22 festgeklebt wird. Das streifenförmige in der selbstklebenden Hüllfolie 24 aufgenommene Dichtungsmaterial 22 dient somit sowohl zur Befestigung der Folie 18 auf der Hautoberfläche 10 als auch zur Abdichtung der Folie 18 an ihrem gesamten Rand. Die Folie 18 kann dabei lediglich zur Abdeckung und zum Schutz des Wundgebietes 12 aufgeklebt werden, wie dies bei der Notfallversorgung häufig wichtig ist. Selbstverständlich kann auch hierbei eine Drainage 16 mittels des Dichtungsmaterials 22 abgedichtet unter die Folie 18 geführt werden.

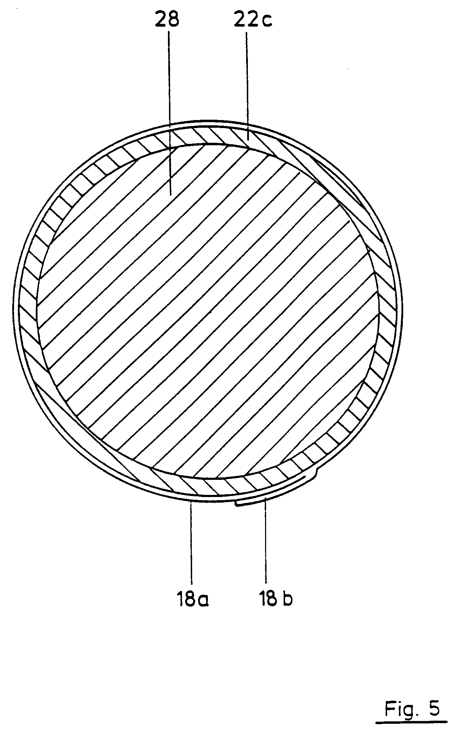

In Figur 5 ist ein Anwendungsbeispiel dieser Ausführung gezeigt. Um eine großflächige Verletzung einer Extremität 28, z.B. eines Beines, in der Notfallversorgung provisorisch zu versiegeln, wird eine sackförmige oder schlauchförmige Folie 18a über den verletzten Bereich der Extremität 28 gezogen. Oberhalb bzw. oberhalb und unterhalb des verletzten Bereiches der Extremität 28 wird ein bandförmiger Streifen 22c des Dichtungsmaterials 22 um die Extremität 28 geklebt. Der offene Rand der sack- oder schlauchförmigen Folie 18a wird um den Streifen 22c des Dichtungsmaterials 22 gespannt und an diesem festgeklebt. Die verbleibende Lose 18b der Folie 18a wird umgeschlagen und mittels einer Binde oder dgl. fixiert und verschlossen. Der Streifen 22c des Dichtungsmaterials 22 gewährleistet eine Abdichtung zwischen Extremität 28 und Folie 18a über den ganzen Umfang der Extremität 28 trotz Unebenheiten und Wölbungen der Hautoberfläche 10.

## Patentansprüche

1. Vorrichtung zum Abdecken eines Wundgebietes (12) mit einer das Wundgebiet (12) überdeckenden wasserdampfdurchlässigen Folie (18), die mit ihrem Rand rings um das Wundgebiet (12) adhäsiv an der Hautoberfläche (10) befestigbar ist, dadurch gekennzeichnet, daß zum Versiegeln des Wundgebietes (12) die Folie (18) luftundurchlässig ist, daß zum luftdichten Verschließen von Undichtigkeiten zwischen der Folie (18) und der Hautoberfläche (10) ein zumindest in einem Bereich des Randes der Folie (18) zwischen der Folie (18) und der Hautoberfläche (10) anzuordnendes leicht verformbares, nicht fließendes Silikon, Hydrokolloid oder Lyogel, insbesondere Hydrogel als Dichtungsmaterial (22) vorgesehen ist und daß das Dichtungsmaterial (22) in Form von mit einer Hüllfolie (24) ummantelten Streifen oder Platten konfektioniert zur Verfügung gehalten wird.

2. Vorrichtung nach Anspruch 1, gekennzeichnet durch eine unter dem Dichtungsmaterial (22) auf die Hautoberfläche (10) klebbare Schutzfolie (20).

3. Vorrichtung nach Anspruch 1, dadurch gekennzeichnet, daß die Hüllfolie (24) eine adhäsive Außenbeschichtung aufweist.

4. Vorrichtung nach Anspruch 3, dadurch gekennzeichnet, daß die Außenbeschichtung mit einer abziehbaren Deckfolie (26) abgedeckt ist.

5. Vorrichtung nach einem der Ansprüche 1 bis 4, dadurch gekennzeichnet, daß im Wundgebiet (12) eine Drainage (16) unter der Folie (18) angeordnet ist, daß die Drainage (16) auf der Hautoberfläche (10) zwischen der Folie (18) und der Hautoberfläche (10) herausgeführt ist und daß die Drainage (16) im Bereich des Randes der Folie (18) von dem Dichtungsmaterial (22) umschlossen ist.

6. Vorrichtung nach Anspruch 5, dadurch gekennzeichnet, daß die Drainage (16) mit dem umschließenden Dichtungsmaterial (22) und der das Dichtungsmaterial (22) ummantelnden Hüllfolie (24) konfektioniert ist.

7. Vorrichtung nach einem der Ansprüche 1 bis 4, dadurch gekennzeichnet, daß das Dichtungsmaterial (22) als bandförmiger Streifen entlang dem gesamten Rand der Folie (18) zwischen der Hautoberfläche (10) und der Folie (18) angeordnet ist.

8. Vorrichtung nach Anspruch 7, dadurch gekennzeichnet, daß der bandförmige Streifen des Dichtungsmaterials (22) von einer beidseitigen selbstklebenden Hüllfolie (24) ummantelt ist.

9. Vorrichtung nach Anspruch 7 oder 8, dadurch gekennzeichnet, daß die Folie (18) schlauch- oder sackförmig einen Körperabschnitt des Patienten umhüllt und daß der bandförmige Streifen des Dichtungsmaterials (22) diesen Körperabschnitt im Bereich des Randes der Folie (18) umschließt.

## Claims

1. A device for covering a wound area (12) with a water-vapour-permeable film (18) covering the wound area (12), which with its edge can be attached adhesively to the skin surface (10) around the wound area (12),
**characterised in that** to seal the wound area (12) the film (18) is air-permeable,
**in that** provided as the sealing material (22) for the air-tight closure of leaks between the film (18) and the skin surface (10) is an easily deformable, non-flowing silicon, hydrocolloid or lyogel, in particular hydrogel, which is to be disposed at least in a region of the edge of the film (18) between the film (18) and the skin surface (10), **and in that** the sealing material (22) is kept available produced in the form of strips or plates encased with an enveloping film (24).

2. A device according to Claim 1,
**characterised** by a protective film (20) which can be stuck to the skin surface (10) beneath the sealing material (22).

3. A device according to Claim 1,
**characterised in that** the enveloping film (24) has an adhesive outer coating.

4. A device according to Claim 3,
**characterised in that** the outer coating is covered, with a pull-off covering film (26).

5. A device according to one of Claims 1 to 4,
**characterised in that** a drain (16) is disposed in the wound area (12) under the film (18),
**in that** the drain (16) is passed out on the skin surface (10) between the film (18) and the skin surface (10)
**and in that** the drain (16) is surrounded by the sealing material (22) in the region of the edge of the film (18).

6. A device according to Claim 5,
**characterised in that** the drain (16) is fabricated with the surrounding sealing material (22) and the enveloping film (24) encasing the sealing material (22).

7. A device according to one of Claims 1 to 4,
**characterised in that** the sealing material (22) is disposed as a ribbon-shaped strip along the entire edge of the film (18) between the skin surface (10) and the film (18).

8. A device according to Claim 7,
**characterised in that** the ribbon-shaped strip of the sealing material (22) is encased by a double-sided self-adhesive film (24).

9. A device according to claim 7 or 8,
**characterised in that** the film (18) envelopes a body portion of the patient in a hose shape or bag shape **and in that** the ribbon-shaped strip of the sealing material (22) encloses this body portion in the region of the film (18).

## Revendications

1. Dispositif pour recouvrir une zone de plaie (12) avec une feuille (18) perméable à la vapeur d'eau recouvrant la zone de plaie (12), feuille susceptible d'être fixée par son bord tout autour de la zone de plaie (12), de façon adhésive, sur la surface de peau (10),
caractérisé en ce que
- pour sceller la zone de plaie (12), la feuille (18) est imperméable à l'air, en ce que pour obtenir une fermeture, étanche à l'air, des sites à défauts d'étanchéité existants entre la feuille (18) et la surface de peau (10) est prévu un silicone, un hydrocolloïde ou un lyogel, en particulier un hydrogel, qui soient facilement déformables, non coulants, à titre de matériau d'étanchéité (22), devant être disposé au moins dans une zone de la bordure de la feuille (18), entre la feuille (18) et la surface de peau (10), et
- le matériau d'étanchéité (22) est maintenu à disposition, confectionné, sous la forme de bandes ou de plaques enrobées par une feuille d'enveloppement (24).

2. Dispositif selon la revendication 1,
caractérisé par
une feuille protectrice (20) susceptible d'être collée sur la surface de peau (10) sous le matériau d'étanchéité (22).

3. Dispositif selon la revendication 1,
caractérisé en ce que
la feuille d'enveloppement (24) présente un revêtement extérieur adhésif.

4. Dispositif selon la revendication 3,
caractérisé en ce que
le revêtement extérieur est recouvert d'une feuille de recouvrement (26) extractible.

5. Dispositif selon l'une des revendications 1 à 4,
caractérisé en ce que
dans la zone de plaie (12) est ménagé sous la feuille (18) un drainage (16), en ce que le drainage (16) est sorti de la surface de peau (10) entre la feuille (18) et la surface de peau (10), et en ce que le drainage (16) est enclos par le matériau d'étanchéité (22) dans la zone de la bordure de feuille (18).

6. Dispositif selon la revendication 5,
caractérisé en ce que
le drainage (16) est confectionné avec le matériau d'étanchéité (22) assurant l'enfermement et avec la feuille d'enveloppement (24) entourant le matériau d'étanchéité (22).

7. Dispositif selon l'une des revendications 1 à 4,
caractérisé en ce que
le matériau d'étanchéité (22) est disposé sous la forme d'une bande en forme de ruban le long de l'ensemble de la bordure de la feuille (18) entre la surface de peau (10) et la feuille (18).

8. Dispositif selon la revendication 7,
caractérisé en ce que
la bande en forme de ruban du matériau d'étanchéité (22) est enveloppée par une feuille d'enveloppement (24), auto-adhésive sur les deux faces.

9. Dispositif selon la revendication 7 à 8,
caractérisé en ce que
- la feuille (18) enveloppe, sous forme de tuyau ou de sac, un tronçon corporel du patient, et
- la bande en forme de ruban du matériau d'étanchéité (22) enferme ce tronçon corporel dans la zone de la bordure de la feuille (18).
